(19) Europäisches Patentamt
European Patent Office
Office européen des brevets

(11) EP 2 259 812 B1

(12) EUROPÄISCHE PATENTSCHRIFT

(45) Veröffentlichungstag und Bekanntmachung des Hinweises auf die Patenterteilung:
**09.11.2016 Patentblatt 2016/45**

(21) Anmeldenummer: **09724844.7**

(22) Anmeldetag: **24.03.2009**

(51) Int Cl.:
***A61M 1/36*** *(2006.01)*

(86) Internationale Anmeldenummer:
**PCT/EP2009/002129**

(87) Internationale Veröffentlichungsnummer:
**WO 2009/118145 (01.10.2009 Gazette 2009/40)**

(54) **VERFAHREN UND VORRICHTUNG ZUR ÜBERWACHUNG EINES GEFÄSSZUGANGS SOWIE EXTRAKORPORALE BLUTBEHANDLUNGSVORRICHTUNG MIT EINER VORRICHTUNG ZUR ÜBERWACHUNG EINES GEFÄSSZUGANGS**

METHOD AND DEVICE FOR MONITORING A VASCULAR ACCESS AND EXTRACORPOREAL BLOOD TREATMENT DEVICE COMPRISING A DEVICE FOR MONITORING A VASCULAR ACCESS

PROCÉDÉ ET DISPOSITIF DE SURVEILLANCE D'UN ACCÈS VASCULAIRE ET SYSTÈME DE TRAITEMENT SANGUIN EXTRACORPOREL ÉQUIPÉ D'UN DISPOSITIF DE SURVEILLANCE D'UN ACCÈS VASCULAIRE

(84) Benannte Vertragsstaaten:
**AT BE BG CH CY CZ DE DK EE ES FI FR GB GR HR HU IE IS IT LI LT LU LV MC MK MT NL NO PL PT RO SE SI SK TR**
Benannte Erstreckungsstaaten:
**RS**

(30) Priorität: **27.03.2008 DE 102008015832**

(43) Veröffentlichungstag der Anmeldung:
**15.12.2010 Patentblatt 2010/50**

(73) Patentinhaber: **Fresenius Medical Care Deutschland GmbH**
**61352 Bad Homburg v.d.H. (DE)**

(72) Erfinder: **Kopperschmidt, Pascal**
**97456 Dittelbrunn (DE)**

(74) Vertreter: **Oppermann, Frank et al**
**OANDO Oppermann & Oppermann LLP**
**Washingtonstrasse 75**
**65189 Wiesbaden (DE)**

(56) Entgegenhaltungen:
**DE-A1-102006 032 815 US-B1- 6 221 040**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 259 812 B1

## Beschreibung

Die Erfindung betrifft ein Verfahren zur Überwachung eines Gefäßzugangs während einer extrakorporalen Blutbehandlung, insbesondere einer chronischen Blutreinigungstherapie wie Hämodialyse, Hämofiltration und Hämodiafiltration, und eine Vorrichtung zur Überwachung eines Gefäßzugangs für eine extrakorporale Blutbehandlungsvorrichtung, insbesondere zur Hämodialyse, Hämofiltration und Hämodiafiltration. Darüber hinaus betrifft die Erfindung eine extrakorporale Blutbehandlungsvorrichtung mit einer Vorrichtung zur Überwachung des Gefäßzugangs.

Bei den bekannten Methoden der chronischen Blutreinigungstherapie wie Hämodialyse, Hämofiltration und Hämodiafiltration wird Blut eines Patienten über einen extrakorporalen Blutkreislauf geleitet. Als Zugang zum Gefäßsystem des Patienten werden arteriovenöse Fisteln, Gefäßimplantate oder auch verschiedene Katheter verwendet. Die Verbindung des Patienten mit dem extrakorporalen Blutkreislauf erfolgt im Allgemeinen über Kanülen, mit denen die Fistel bzw. das Gefäßimplantat punktiert wird.

Falls sich während der Blutbehandlung der Anschluss zwischen dem extrakorporalen Blutkreislauf und dem Gefäßsystem löst bzw. ein Blutleck im extrakorporalen Kreislauf auftritt, kann ein Verbluten des Patienten nur verhindert werden, wenn der extrakorporale Blutfluss sofort gestoppt wird. Daher sind extrakorporale Blutkreisläufe in der Regel mit Schutzsystemen ausgestattet, die permanent den arteriellen und venösen Druck ($P_A$ und $P_V$) innerhalb des Systems sowie den Eintritt von Luft in den extrakorporalen Kreislauf überwachen.

Es sind druckbasierende maschinenseitige Schutzsysteme bekannt, die für den Fall, dass sich die Verbindung zwischen dem Patienten und dem arteriellen Zweig des extrakorporalen Kreislaufs löst, schnell ansprechen. Für den Fall jedoch, dass sich die venöse Kanüle aus dem Gefäßzugang löst, ist ein Ansprechen der bekannten druckbasierenden Schutzsysteme nicht immer sichergestellt. Auch im Falle eines Blutlecks im venösen Schlauchsystem kann es vorkommen, dass der resultierende venöse Druckabfall nicht ausreicht, um eine Auslösen der Schutzsysteme zu gewährleisten.

Die US 6,221,040 B1 beschreibt eine Vorrichtung zur Überwachung eines Gefäßzugangs, mit der sowohl das Herausrutschen der arteriellen Kanüle als auch der venösen Kanüle sicherer erkannt werden kann. Zur Überwachung des Gefäßzugangs wird der Druck sowohl im arteriellen als auch im venösen Zweig des extrakorporalen Kreislaufs mittels Drucksensoren überwacht. Aus dem arteriellen und venösen Druck werden für den Zustand des Gefäßzugangs charakteristische Werte in einer Recheneinheit berechnet, die in einer Auswerteinheit zur Erkennung eines fehlerhaften Gefäßzugangs ausgewertet werden. Zur Berechnung der für den Zustand des Gefäßzugangs charakteristischen Werte können die Summe und die Differenz des venösen und arteriellen Drucks im extrakorporalen Kreislauf bestimmt werden.

Neben dem obigen Verfahren, bei dem der Druck im arteriellen und venösen Zweig des extrakorporalen Blutkreislaufs überwacht wird, sind auch Überwachungsvorrichtungen bekannt, die auf der Überwachung von sich im extrakorporalen Kreislauf fortpflanzenden Druckpulsen beruhen.

Die DE 101 15 991 C1 (US 2002/0174721 A1) beschreibt ein Verfahren zum Erkennen von Stenosen in einem Schlauchleitungssystem während einer extrakorporalen Blutbehandlung, bei dem ein oszillierendes Drucksignal in dem Schlauchleitungssystem generiert und das oszillierende Drucksignal gemessen wird. Zum Erkennen von Stenosen wird das Frequenzspektrum des oszillierenden Drucksignals analysiert, wobei auf eine Stenose bei einer Änderung des Frequenzspektrums geschlossen wird. Hierzu wird eine Fouriertransformation des oszillierenden Drucksignals im venösen Zweig des extrakorporalen Kreislaufs vorgenommen. Aus dem Fourierspektrum des venösen Drucksignals wird der statische Anteil extrahiert, wobei die Dämpfung mindestens einer Oberschwingung des Drucksignals ermittelt und aus der Änderung der Dämpfung auf eine Stenose geschlossen wird. Aus der DE 10 2006 032 815 A1 ist eine Vorrichtung zur Überwachung des Gefäßzugangs für eine extrakorporale Blutbehandlungsvorrichtung bekannt, bei der Blut aus dem Gefäßzugang über einen arteriellen Zweig eines extrakorporalen Blutkreislaufs in einen Dialysator strömt und aus dem Dialysator über einen venösen Zweig zurück in den Gefäßzugang strömt. Die bekannte Vorrichtung sieht eine Messeinheit zum Messen des Drucks im venösen und arteriellen Zweig des extrakorporalen Blutkreislaufs und eine Auswerteinheit zum Auswerten des gemessenen venösen und arteriellen Drucks vor, um einen fehlerhaften Gefäßzugang feststellen zu können. Die Auswerteinheit ermittelt zur Erkennung des fehlerhaften Gefäßzugangs aus dem gemessenen venösen und arteriellen Druck einen Differenzdruck.

Der Erfindung liegt die Aufgabe zu Grunde, ein Verfahren zur Überwachung eines Gefäßzugangs während einer extrakorporalen Blutbehandlung anzugeben, das einen relativ geringen apparativen Aufwand erfordert, um mit hoher Sicherheit einen fehlerhaften Gefäßzugang sowohl auf der arteriellen als auch der venösen Seite des extrakorporalen Blutkreislaufs erkennen zu können.

Eine weitere Aufgabe der Erfindung liegt darin, eine Vorrichtung zur sicheren Überwachung des arteriellen und venösen Gefäßzugangs für eine extrakorporale Blutbehandlungsvorrichtung zu schaffen, welche einen relativ geringen apparativen Aufwand erfordert.

Darüber hinaus ist eine Aufgabe der Erfindung, eine extrakorporale Blutbehandlungsvorrichtung bereitzustellen, die eine Überwachung des arteriellen und venösen Gefäßzugangs mit hoher Sicherheit und verhältnismäßig geringem apparativen Aufwand ermöglicht.

Die Lösung dieser Aufgaben erfolgt erfindungsgemäß mit den Merkmalen der Patentansprüche 1, 8 und 15.

Vorteilhafte Ausführungsformen der Erfindung sind Gegenstand der Unteransprüche.

Das erfindungsgemäße Verfahren und die erfindungsgemäße Vorrichtung beruhen auf der Überwachung der Differenz zwischen dem venösen Druck im venösen Zweig und dem arteriellen Druck im arteriellen Zweig des extrakorporalen Blutkreislaufs, der nachfolgend auch als venöser und arterieller Differenzdruck bezeichnet wird. Zur Messung des arteriellen und venösen Drucks kann bei dem erfindungsgemäßen Verfahren und der erfindungsgemäßen Vorrichtung von den Sensoren Gebrauch gemacht werden, die in den bekannten Blutbehandlungsvorrichtungen bereits vorhanden sind. Somit kann sich die maschinenseitige Änderung zur Implementierung des erfindungsgemäßen Schutzsystems lediglich auf eine Modifikation der Maschinensteuerung beschränken.

Der Erfindung liegt die Erkenntnis zu Grunde, dass die gemessenen Werte für den venösen und arteriellen Druck von Störsignalen überlagert sind, die eine sichere Auswertung der gemessenen Drucksignale für die Erkennung eines fehlerhaften Gefäßzugangs allein auf der Grundlage des Differenzdrucks grundsätzlich schwierig gestalten. Die Drucksignale werden beispielsweise durch die im extrakorporalen Blutkreislauf angeordnete Blutpumpe, die Ultrafiltrationspumpe sowie Ventile oder dergleichen moduliert.

Bei dem erfindungsgemäßen Verfahren und der erfindungsgemäßen Vorrichtung wird eine die Störungen im extrakorporalen Blutkreislauf beschreibende Testfunktion ermittelt. Mit der Testfunktion wird aus dem gemessenen venösen und arteriellen Druck ein von Störungen befreiter Differenzdruck ermittelt, der zur Erkennung eines fehlerhaften Gefäßzugangs ausgewertet wird.

Die Ermittlung der Störungen im extrakorporalen Blutkeislauf beschreibenden Testfunktion ist grundsätzlich auch dann vorteilhaft, wenn zur Erkennung eines fehlerhaften Gefäßzugangs nur der venöse Druck im extrakorporalen Kreislauf überwacht wird. Von Vorteil ist aber die Berücksichtigung sowohl des venösen als auch arteriellen Drucks, da sich mit einer Überwachung des Gefäßzugangs auf der Grundlage des Differenzdrucks Veränderungen des hydrostatischen Drucks im extrakorporalen Kreislauf kompensieren lassen, die auf Lageänderungen des Patientenzugangs zurückzuführen sind.

Die eigentliche Auswertung des von Störungen befreiten Differenzdrucks zur Erkennung eines fehlerhaften Gefäßzugangs kann an die unterschiedlichsten Kriterien geknüpft sein. Für die Erfindung ist allein entscheidend, dass ein mittels der Testfunktion von Störungen befreiter Differenzdruck ausgewertet wird.

Die Ermittlung des von Störungen befreiten Differenzdrucks kann dadurch erfolgen, dass das arterielle und venöse Drucksignal zunächst einzeln entstört werden, bevor der Differenzdruck gebildet wird. Es ist aber auch möglich, zunächst den Differenzdruck zu bilden, um dann den Differenzdruck zu entstören.

Wenn in den Ansprüchen also von der Bestimmung einer Störungen des arteriellen und venösen Drucks beschreibenden Testfunktion die Rede ist, soll dies sowohl den Fall einer "einfachen" Entstörung mit einer Testfunktion nach der Bildung des Differenzdrucks als auch den Fall einer "zweifachen" Entstörung mit zwei Testfunktionen vor der Differenzbildung einschließen. Es sind also die folgenden Fälle möglich.

Der venöse Druck $P_V$ wird mit der Testfunktion $\hat{P}_V$ und der arterielle Druck $P_A$ mit der Testfunktion $\hat{P}_A$ entstört, und aus beiden entstörten Signalen wird die Differenz gebildet. Folglich gibt es zwei Testfunktionen.

Die Testfunktion $\hat{P}_V$ wird anhand von $P_V$ ermittelt, die Testfunktion $\hat{P}_A$ anhand von $P_A$, wobei $P_V$ - $P_A$ dann mit $\hat{P}_V$ - $\hat{P}_A$ entstört wird. Dieser Fall ist zu dem ersten Fall äquivalent, wobei es aber nur eine Testfunktion gibt.

Die Differenz von venösem und arteriellem Druck $P_V$ - $P_A$ wird mit der Testfunktion

$$\widehat{(P_V - P_A)}$$

entstört.

Bei einer bevorzugten Ausführungsform, die sich durch einen verhältnismäßig geringen Aufwand bei der Berechnung der einzelnen Größen auszeichnet, werden nicht der arterielle und venöse Druck vor der Differenzbildung von Störungen befreit, sondern zunächst wird die Differenz des venösen und arteriellen Drucks gebildet und erst nach der Differenzbildung erfolgt die Befreiung des zuvor ermittelten Differenzdrucks mit der Testfunktion von den Störungen. Durch die Vermeidung einer separaten Entstörung der Operanden vor der Differenzbildung kann der erforderliche Rechenaufwand verhältnismäßig gering gehalten werden.

Der Erfindung liegt die Erkenntnis zu Grunde, dass die Störungen im Wesentlichen periodischer Natur sind. Daher kann die Testfunktion aus einer Linearkombination von trigonometrischen Funktionen generiert werden, wobei die trigonometrischen Funktionen die Voraussetzungen der Orthogonalität erfüllen. Eine derartige Testfunktion erlaubt die Entstörung des Differenzdrucks nach der Differenzbildung.

Bei der Störungen des gemessenen venösen und arteriellen Drucks beschreibenden Testfunktion handelt es sich um eine Funktion, die in einem bestimmten Zeitintervall während der extrakorporalen Blutbehandlung den gemessene Differenzdruck beschreibt, wobei angenommen wird, dass in diesem Zeitintervall der Gefäßzugang ordnungsgemäß ist, d.h. der Differenzdruck nur von den Störungen im System beschrieben wird. Dies kann beispielsweise dadurch

sichergestellt werden, dass der Gefäßzugang vom Arzt auf seine Ordnungsmäßigkeit überprüft wird.

In einem nachfolgenden Zeitintervall wird dann die in dem vorausgehenden Zeitintervall ermittelte Testfunktion herangezogen, um den in diesem Zeitintervall gemessenen Druckdifferenz von den Störungen zu befreien. Die in dem vorausgehenden Zeitintervall ermittelte Testfunktion wird somit als eine Störungen des venösen und arteriellen Drucksignals beschreibende Schätzfunktion für das nachfolgende Zeitintervall angenommen. Dies trifft insbesondere dann zu, wenn die aufeinander folgenden Zeitintervalle unmittelbar aufeinander folgen. Daher sieht die Erfindung eine möglichst zeitnahe Ermittelung der Testfunktion vor.

Bei einer bevorzugten Ausführungsform wird die Testfunktion sukzessive während der extrakorporalen Blutbehandlung ermittelt und während der Blutbehandlung laufend optimiert.

Die Ermittlung der Testfunktion kann grundsätzlich mit unterschiedlichen Algorithmen erfolgen, die dem Fachmann bekannt sind. Vorzugsweise werden die Koeffizienten der Testfunktion nach dem Verfahren der kleinsten Quadrate ermittelt, das dem Fachmann bekannt ist.

Eine bevorzugte Ausführungsform der Erfindung sieht im einfachsten Fall einen Vergleich des entstörten Differenzdrucks mit einem vorgegebenen Grenzwert vor, wobei auf einen fehlerhaften Gefäßzugang dann geschlossen wird, wenn der Differenzdruck kleiner als ein vorgegebener Grenzwert ist. Es können aber auch andere Kriterien für die Auswertung des entstörten Differenzdrucks herangezogen werden, die auch miteinander kombiniert werden können.

Im Folgenden wird das erfindungsgemäße Verfahren zur Überwachung eines Gefäßzugangs sowie eine Vorrichtung zur extrakorporalen Blutbehandlung mit einer Vorrichtung zur Überwachung des Gefäßzugangs unter Bezugnahme auf die Zeichnungen anhand eines Ausfuhrungsbeispiels näher erläutert.

Es zeigen:

Fig. 1 die wesentlichen Komponenten einer Hämodialysevorrichtung zusammen mit der erfindungsgemäßen Vorrichtung zur Überwachung eines Gefäßzugangs in stark vereinfachter schematischer Darstellung,

Fig. 2 einen Korrekturfaktor zur Korrektur des Differenzdrucks als Funktion des arteriellen Drucks und

Fig. 3 die gestörten und entstörten Drucksignale als Funktion der Zeit.

Fig. 1 zeigt eine vereinfachte schematischer Darstellung einer Blutbehandlungsvorrichtung mit einer Vorrichtung zur Überwachung des arteriellen und venösen Gefäßzugangs, die über ein Blutbehandlungselement verfügt, bei dem es sich um einen Dialysator, Filter Adsorber, Oxygenerator oder eine Blutzentrifuge handeln kann. Im vorliegenden Ausführungsbeispiel handelt es sich bei der Blutbehandlungsvorrichtung um eine Dialysevorrichtung, die über einen Dialysator als Blutbehandlungslement verfügt.

Die Überwachungsvorrichtung kann eine selbständige Einheit bilden, aber auch Bestandteil der Dialysevorrichtung sein. Vorzugsweise ist die Überwachungsvorrichtung Bestandteil der Dialysevorrichtung, da einzelne Komponenten der Überwachungsvorrichtung bereits in der Dialysevorrichtung vorhanden sind. Beispielsweise kann die Überwachungsvorrichtung von dem in einer Dialysevorrichtung ohnehin vorhandenen Drucksensoren, aber auch von der Steuer- und Recheneinheit der Dialysevorrichtung Gebrauch machen.

Die Dialysevorrichtung weist einen Dialysator 1 auf, der durch eine semipermeable Membran 2 in eine Blutkammer 3 und eine Dialysierflüssigkeitskammer 4 unterteilt ist. An dem Einlass der Blutkammer 3 ist eine arterielle Blutleitung 5 angeschlossen, in die eine peristaltische Blutpumpe 6 geschaltet ist. Stromab der Blutkammer 3 führt eine venöse Blutleitung 7 von dem Auslass der Blutkammer zu dem Patienten. In die venöse Blutleitung 7 ist eine Tropfkammer 8 geschaltet. An den Enden der arteriellen und venösen Blutleitung 5, 7 sind Kanülen 5a, 7a angeschlossen, die in den Patienten gestochen werden. Die arterielle und venöse Blutleitung sind Bestandteil eines als Disposable ausgebildeten Schlauchleitungssystems.

In einer Dialysierflüssigkeitsquelle 9 wird frische Dialysierflüssigkeit bereitgestellt. Von der Dialysierflüssigkeitsquelle 9 führt eine Dialysierflüssigkeitszuführleitung 10 zu dem Eingang der Dialysierflüssigkeitskammer 4 des Dialysators, während eine Dialysierflüssigkeitsabführleitung 11 von dem Ausgang der Dialysierflüssigkeitskammer zu einem Abfluss führt. Die Dialysierflüssigkeitspumpe zum Fördern der Dialysierflüssigkeit ist in Fig. 1 nicht dargestellt.

Zur Unterbrechung des Blutflusses ist an der venösen Blutleitung 7 stromab der Tropfkammer 8 eine Absperrklemme 13 vorgesehen, die elektromagnetisch betätigt wird. Die arterielle Blutpumpe 6 und die venöse Absperrklemme 13 werden über Steuerleitungen 14, 15 von einer zentralen Steuer- und Recheneinheit 16 der Dialysevorrichtung angesteuert.

Die Dialysevorrichtung kann noch über weitere Komponenten, beispielsweise eine Bilanziereinrichtung und eine Ultrafiltrationseinrichtung etc., verfügen, die aber der besseren Übersichtlichkeit halber in Fig. 1 nicht dargestellt sind.

Die Vorrichtung 17 zur Überwachung des Gefäßzugangs weist eine Messeinheit 18, 19 auf, die einen den Druck in der arteriellen Blutleitung 5 überwachenden arteriellen Drucksensor 18 und einen den Druck in der venösen Blutleitung 7 überwachenden venösen Drucksensor 19 aufweist. Die Messwerte der Drucksensoren 18, 19 werden über

Datenleitungen 20, 21 an eine Rechen- und Auswerteinheit 22 der

Überwachungsvorrichtung 17 übertragen, in der die Messwerte zur Erkennung eines fehlerhaften Gefäßzugangs ausgewertet werden. Die Rechen- und Auswerteinheit 22 der Überwachungsvorrichtung 17 kann auch Bestandteil der zentralen Steuer- und Recheneinheit 16 der Dialysevorrichtung sein.

In einer Speichereinheit 23, die über eine Datenleitung 24 mit der Rechen- und Auswerteinheit 22 verbunden ist, werden die bei der Berechnung anfallenden Zwischenergebnisse abgelegt.

An der Recheneinheit 22 der Überwachungsvorrichtung 17 ist über eine Steuerleitung 26 eine Alarmeinheit 25 angeschlossen, die über eine weitere Steuerleitung 27 mit der zentralen Steuer- und Recheneinheit 16 der Dialysevorrichtung verbunden ist.

Nachfolgend werden die theoretischen Grundlagen für die Drucküberwachung und die Funktionsweise der erfindungsgemäßen Überwachungsvorrichtung im Einzelnen beschrieben.

Der maschinenseitig gemessene venöse Druck in der venösen Blutleitung 7 setzt sich bei rotierender Blutpumpe 6 im Allgemeinen aus dem Staudruck der venösen Punktionskanüle 7a, dem Fistelinnendruck und einem hydrostatischen Druck zusammen, der von der geometrischen Höhendifferenz zwischen dem venösen Drucksensor 19 und dem Herz des Patienten abhängt und sich bei vertikaler Lageänderung des Patienten ändert. Zu dem venösen Druck trägt im Wesentlichen der Staudruck vor der venösen Kanüle 7a bei. Das Drucksignal des venösen Drucksensors 19 ist im Allgemeinen nicht stationär, sondern wird durch die Blutpumpe 6 und die nicht dargestellte Ultrafiltrationspumpe sowie hydraulische Ventile und andere nicht dargestellte Komponenten der Dialysevorrichtung mit periodischen Störsignalen überlagert.

Der Fistelinnendruck, der nur geringfügig zum venösen Druck beiträgt, setzt sich aus dem mittleren Blutdruck und dem durch das im Allgemeinen venöse Gefäßsystem verursachten rheologischen Druckabfall stromab der Punktionsstelle zusammen. Der durch den Fistelinnendruck gewonnene Beitrag zum maschinenseitig gemessenen venösen Druck liegt bei etwa 8 bis 10 % und variiert in Abhängigkeit von der Art des Patientenzugangs zwischen 15 und 35 mmHg.

Bei einer Dislokation der venösen Punktionsnadel reduziert sich der venöse Druck um den Beitrag des Fistelinnendrucks. Die bekannten Überwachungssysteme können aber die auf einen fehlerhaften Gefäßzugang zurückzuführende Reduzierung des Fistelinnendrucks zum Teil nicht erkennen, da die untere Überwachungsgrenze für den venösen Druck im Allgemeinen nicht unterschritten wird. Die Erfindung erlaubt jedoch die Erkennung der Reduzierung des Fistelinnendrucks in Folge eines fehlerhaften Gefäßzugangs mit höherer Sicherheit, indem die störenden Beiträge zum venösen Druck eliminiert werden. Der so stabilisierte venöse Druck behält seine Dynamik bei und kann als Messgröße zur Überwachung des Gefäßzugangs während der Dialyse genutzt werden.

Für den Fall einer Diskonnektion der venösen Kanüle reduziert sich der von der periodischen Störung befreite venöse Druck, während der maschinenseitig gemessene arterielle Druck weitgehend unverändert bleibt. Daher ist grundsätzlich eine Berücksichtigung des arteriellen Drucks nicht erforderlich. Auf Grund von Lageänderungen des Patienten kann es aber zur Veränderung des hydrostatischen Drucks kommen. Daher sieht die Erfindung vor, dass nicht allein der venöse Druck, sondern die Differenz zwischen dem venösen und arteriellen Druck überwacht wird.

Wenn die Lage der Punktionsstelle verändert wird, beispielsweise wenn der Patient seinen Arm hebt oder der Patient aufsteht, wird durch die Bestimmung des Differenzdrucks der Einfluss einer Veränderung des hydrostatischen Drucks kompensiert.

Da ein sich ändernder arterieller Druck im Allgemeinen zu einer Änderung des Querschnitts des von der vorzugsweise peristaltischen Blutpumpe abgehenden Blutschlauchsegments führt, reduzieren sich bei fallendem arteriellen Druck die effektive Blutförderrate und somit auch die Staudrücke in der arteriellen oder venösen Punktionskanüle 5a, 7a. Daher führt eine vertikale Lageänderung des Patienten zu leicht unterschiedlichen Reaktionen an den arteriellen und venösen Druckmessstellen, die vom absoluten arteriellen Druck abhängig sind. Diese Reaktionen können bei der Erfindung berücksichtigt werden, wie nachfolgend noch erläutert wird.

Während der extrakorporalen Blutbehandlung werden die Messwerte ($P_A$ und $P_V$) des arteriellen und venösen Drucksensors 18, 19 der Messeinheit fortlaufend erfasst, in der Rechen- und Auswerteinheit 22 über eine Halbperiode der Blutpumpe 6 gemittelt und in der Speichereinheit 23 gespeichert. Auf Grund der unterschiedlichen Ankopplung des arteriellen und venösen Drucksensors 18, 19 an das Blutschlauchsystem 5, 7 kann eine Anpassung der Dynamik der Sensoren erforderlich sein. Dies erfolgt vorzugsweise mit einer Tiefpassfilterung des mit dem arteriellen Drucksensor 18 gemessenen Drucks, die in der Rechen- und Auswerteinheit 22 erfolgt.

Zur Kompensation der Drucksignale in Folge der angesprochenen Lageänderungen des Patienten, die zu hydrostatischen Druckänderungen an den Druckmessstellen führen, bestimmt die Rechen- und Auswerteinheit einen Korrekturfaktor $\kappa$ als Funktion des arteriellen Drucks $P_A$. Fig. 2 zeigt ein Beispiel einer funktionalen Abhängigkeit von $\kappa$ ($P_A$). Für $P_A > 0$ braucht im Allgemeinen eine Korrektur nicht zu erfolgen, d.h. in diesem Bereich ist zweckmäßig $\kappa$ ($P_A$) = 100 %.

Die Rechen- und Auswerteinheit 22 überwacht zur Erkennung eines fehlerhaften Gefäßzugangs die kompensierte Druckdifferenz $P_{VA}(t)$ aus dem venösen und arteriellem Druck ($P_V(t)$ und $P_A(t)$), die nach der folgenden Gleichung berechnet wird.

$$P_{VA}(t) = \kappa(P_A)\cdot[P_V(t)-P_A(t)] \quad (1)$$

Diese Funktion kann zweckmäßig zu Zeitpunkten $t = n\pi/\omega_{Qb}$ abgetastet werden, wobei n für die Zahl der Rotorköpfe bzw. Rollen der Blutpumpe 6 und $\omega_{Qb}$ für die Kreisfrequenz der Blutpumpe steht. In diesem Fall liegt unabhängig von der eigentlichen Fördergeschwindigkeit zwischen jeweils zwei Messpunkten der gleiche Drehwinkel der Pumpe und damit eine konstante geförderte Blutmenge. Mit anderen Worten geht dann die Reaktionszeit oder -geschwindigkeit mit einer jeweils bestimmten konstanten Blutmenge einher.

Bei einer venösen Diskonnektion ist somit die Menge des Blutflusses über die diskonnektierte Kanüle unabhängig von der Förderrate der Blutpumpe, so dass die Überwachungskriterien an den Blutfluss gekoppelt sind.

Die Rechen- und Auswerteinheit 22 bestimmt während der extrakorporalen Blutbehandlung weiterhin fortlaufend die nachfolgende Testfunktion.

$$\hat{P}_{VA}(t)=B_0+\sum_{k=1}^{K}\sum_{n=1}^{N}\left[A_{nk}\sin(n\omega_k t)+B_{nk}\cos(n\omega_k t)\right] \quad (2)$$

Die Koeffizienten A und B in Gleichung (2) bestimmen die Beiträge der höheren Harmonischen der Ordnung n der periodischen Störung k mit der Frequenz $\omega_k$. Die Koeffizienten A und B werden mittels des Verfahrens der kleinsten Quadrate während der extrakorporalen Blutbehandlung sukzessive geschätzt. Die Schätzung wird mit jeder Halbrotation der Blutpumpe optimiert. Ein Vergessenheitsfaktor bestimmt die Kopplung zwischen der aktuell erhaltenen Schätzung und der bereits zurückliegenden Schätzung.

In Gleichung (2) gibt der erste Summand $B_0$ den von Störungen befreiten stabilen Differenzdruck an, während der zweite Summand den Störungsbeitrag aller periodischen Teilnehmer angibt. Da sich die Koeffizienten der Beiträge bei erfolgreicher Schätzung nur langsam ändern, kann mittels Gleichung (2) eine Approximation der Druckverhältnisse in der unmittelbaren Zukunft realisiert werden. Die Bestimmung der Testfunktion erfolgt in der Rechen- und Auswerteinheit 22 wie folgt.

Die Testfunktion beschreibt die mit dem Korrekturfaktor korrigierte Druckdifferenz $P_{VA}(t)$ aus dem venösen und arteriellen Druck, der mit dem venösen und arteriellen Drucksensor 18, 19 innerhalb eines Zeitintervalls der Blutbehandlung gemessen wird, in dem davon auszugehen ist, dass ein ordnungsgemäßer Gefäßzugang vorliegt. Da ein fehlerhafter Gefäßzugang in diesem Zeitintervall nicht vorliegt, entspricht der korrigierte Differenzdruck $P_{VA}(t)$ dem von der Testfunktion beschriebenen Druck $\hat{P}_{VA}(t)$, wobei dieser Druck nur den Störanteil und das Nutzsignal ($B_0$) enthält.

Die in dem vorgegebenen Zeitintervall ermittelte Testfunktion $\hat{P}_{VA}(t)$ wird als eine Schätzfunktion für ein nachfolgendes Zeitintervall angenommen, in dem ein fehlerhafter Gefäßzugang erkannt werden soll. Ein fehlerhafter Gefäßzugang wird in dem nachfolgenden Zeitintervall dann erkannt, wenn bei der Gegenüberstellung der kompensierten Druckdifferenz und der Schätzfunktion (Testfunktion) bestimmte in der Rechen- und Auswerteinheit vorgegebene Kriterien erfüllt sind.

Der Ermittlung der Testfunktion nach dem Verfahren der kleinsten Quadrate beruht auf den folgenden theoretischen Grundlagen.

Es wird angenommen, dass die Störungen ein Signal darstellen, das sich aus mehreren periodischen Signalgebern verschiedener Frequenzen zusammensetzt. Dieses Signal soll in eine Reihe harmonischer Funktionen zerlegt werden. Die Zerlegung soll Anteile aller Signalgeber enthalten. Mittels des Verfahrens der kleinsten Quadrate (*"Recursive Least Square* "-Verfahren *RLS*), soll eine Abschätzung der Fourierkoeffizienten erfolgen und eine sukzessive Optimierung der Koeffizienten gelingen.

Sei p(t) das physikalisch gemessene Ausgangssignal zur diskreten Zeiterfassung. Die geschätzte Funktion des Ausgangssignals ist die Summe einer harmonischen Zerlegung d(t) gemäß:

$$d(t)=\sum_{k=0}^{K}\sum_{n=0}^{N}\left[\alpha_{n,k}\sin\left(2\pi n\frac{t}{T_k}\right)+\beta_{n,k}\cos\left(2\pi n\frac{t}{T_k}\right)\right] \quad (3)$$

Die Fourierkoeffizienten der Harmonischen n, des Signalgebers k sind durch $\alpha_{n,k}$ und $\beta_{n,k}$ gegeben. Die entsprechenden Periodendauern sind $T_k$. K ist die Anzahl der Signalgeber, N die Anzahl der beschränkten höheren Harmonischen, die für die Zerlegung als ausreichend angesehen werden. Mittels eines geeigneten Tiefpasses werden die zur Abschätzung benötigten Harmonischen von p(t) limitiert.

Die Koeffizienten der Abschätzung d(t) werden nun bei Kenntnis der Periodendauern der Signalgeber ermittelt,

so dass die Summe der quadratischen Fehler des Abstandes $|p(t)-d(t)|^2$ für eine Anzahl von Zeiterfassungsmomenten t im diskretem Zeitintervall [M-I,M] minimiert ist.

Insbesondere gilt unter Berücksichtigung eines Vergessenheitsfaktors $0<\lambda<1$, der den Einfluss der zeitlich vergangenen quadratischen Distanzen reduziert.

$$\sum_{t'=t}^{t-M} \lambda^{t-t'} \left| p(t') - d(t') \right|^2 = \min \qquad (4)$$

Folglich reduziert sich bei einem konstanten λ das Gewicht, mit dem eine Abweichung zwischen p(t') und d(t') in die Extremwertbetrachtung eingeht, mit der wachsenden Zeitspanne t-t', mit der ein Zeitpunkt t' in der Vergangenheit liegt.

Für die Ermittlung der Testfunktion sind dem Fachmann jedoch auch andere mathematische Verfahren geläufig.

Nach der Ermittlung der Testfunktion (Schätzfunktion) subtrahiert die Rechen- und Auswerteinheit 22 die Approximation der Druckverhältnisse ohne den stabilen Beitrag von dem gemessenen Differenzdruck $P_{VA}(t)$:

$$PP_{VA}(t) = P_{VA}(t)-(\hat{P}_{VA}(t)-B_0) \qquad (5)$$

Der erhaltene Differenzdruck $PP_{VA}(t)$ ist eine in der Dynamik der Messgröße $P_{VA}$ vergleichbare Observable, die jedoch keinen periodischen Störungsanteil besitzt. Diese Größe kann noch von einzelnen Instabilitäten, die aber nicht periodischer Natur sind, befreit werden. Hierzu kann beispielsweise ein Spikefilter eingesetzt werden.

Die Rechen- und Auswerteinheit 22 überprüft während der extrakorporalen Blutbehandlung laufend das nachfolgende Kriterium.

$$[B_0-PP_{VA}(t)] > Crit_0 \qquad (6)$$

Das obige Kriterium ist erfüllt, sobald eine positive Druckänderung des entstörten Differenzdrucks $PP_{VA}$ um $Crit_0$ gegenüber dem sich langsam ändernden stabilen Beitrag $B_0$ erfolgt. Wenn dies der Fall ist, erzeugt die Rechen- und Auswerteinheit 22 ein Alarmsignal, dass die Alarmeinheit 25 über die Steuerleitung 26 empfängt. Daraufhin gibt die Alarmeinheit 25 einen akustischen und/oder optischen Alarm. Weiterhin sendet die Alarmeinheit über die Steuerleitung 27 ein Steuersignal an die Steuer- und Recheneinheit 16 der Dialysevorrichtung, die daraufhin die venöse Absperrklemme 13 schließt und die Blutpumpe 6 anhält, so dass die Blutbehandlung unterbrochen ist.

Zur Veranschaulichung des Verfahrens zeigt Fig. 3 den mit Störungen behafteten Differenzdruck aus venösem Druck und arteriellem Druck als Funktion der Zeit, wobei der venöse Druck $P_V$ und der arterielle Druck $P_A$ mit dem venösen und arteriellen Drucksensor 19, 18 gemessen und der Differenzdruck in der Rechen- und Auswerteinheit 22 aus arteriellem und venösem Druck berechnet wird. Das mit dem venösen Drucksensor 19 gemessene venöse Drucksignal ist in Fig. 3 mit dem Bezugszeichen I bezeichnet, während das mit dem arteriellen Drucksensor 18 gemessene arterielle Drucksignal mit II bezeichnet ist. Der Differenzdruck ist mit III bezeichnet.

Darüber hinaus zeigt Fig. 3 den von Störungen befreiten Differenzdruck als Funktion der Zeit, der mit dem Bezugszeichen IV bezeichnet ist, der aus Darstellungsgründen nicht wie in Gleichung (5) um Bo verschoben ist, sondern die Funktion $PP_{VA}(t) = P_{VA}(t) - \hat{P}_{VA}(t)$ wiedergibt. Für das Signal IV ist die rechte Y-Achse maßgeblich. Der Differenzdruck wurde durch einen Vergleich des gemessenen Differenzdrucksignals mit der Testfunktion (Schätzfunktion) gewonnen. Wenn das gemessene Differenzdrucksignal mit der Testfunktion (Schätzfunktion) identisch ist oder nur geringfügig von dem gemessenen Differenzdrucksignal abweicht, wird ein ordnungsgemäßer Gefäßzugang angenommen. Wenn jedoch ein fehlerhafter Gefäßzugang vorliegt, ist das gemessene Differenzdrucksignal nicht mit der Testfunktion (Schätzfunktion) identisch und weicht um ein vorgegebenes Maß von der Testfunktion ab, die nur die periodischen Störungen bei einem ordnungsgemäßen Gefäßzugang beschreibt. Dies wird durch eine Änderung des von den Störungen befreiten Differenzdrucks (Signal IV) deutlich. Die in Fig. 3 gezeigten Störungen entsprechen dabei zu Testzwecken durchgeführten Manipulationen am Blutschlauchsystem.

## Patentansprüche

1. Verfahren zur Überwachung eines Gefäßzugangs während einer extrakorporalen Blutbehandlung, bei der Blut aus dem Gefäßzugang über einen arteriellen Zweig eines extrakorporalen Blutkreislaufs in ein Blutbehandlungselement

strömt und aus dem Blutbehandlungselement über einen venösen Zweig zurück in den Gefäßzugang strömt, wobei der Druck im venösen und arteriellen Zweig des extrakorporalen Blutkreislaufs gemessen und der gemessene venöse und arterielle Druck zur Feststellung eines fehlerhaften Gefäßzugangs ausgewertet wird,
**dadurch gekennzeichnet,**
**dass** eine Störungen des gemessenen arteriellen und venösen Drucks beschreibende Testfunktion ermittelt wird und **dass** mit der Störungen des arteriellen und venösen Drucks beschreibenden Testfunktion ein von Störungen befreiter Differenzdruck aus dem gemessenen venösen und arteriellen Druck ermittelt wird, und
**dass** der von Störungen befreite Differenzdruck zur Erkennung eines fehlerhaften Gefäßzugangs ausgewertet wird.

**2.** Verfahren nach Anspruch 1, **dadurch gekennzeichnet, dass** zur Ermittlung des von Störungen befreiten Differenzdrucks die Differenz des venösen und arteriellen Drucks gebildet wird und dass nach der Differenzbildung der Differenzdruck mit der Störungen des arteriellen und venösen Drucks beschreibenden Testfunktion von Störungen befreit wird.

**3.** Verfahren nach Anspruch 2, **dadurch gekennzeichnet, dass** die Testfunktion in einem vorausgehendes Zeitintervall ermittelt wird, und dass der gemessene Differenzdruck in einem nachfolgenden Zeitintervall mit der in dem vorausgehenden Zeitintervall ermittelten Testfunktion verglichen wird, wobei die in dem vorausgehenden Zeitintervall ermittelte Testfunktion als eine Störungen des arteriellen und venösen Drucksignals beschreibende Schätzfunktion für das nachfolgende Zeitintervall angenommen wird.

**4.** Verfahren nach Anspruch 3, **dadurch gekennzeichnet, dass** die Testfunktion während der extrakorporalen Blutbehandlung sukzessive ermittelt wird.

**5.** Verfahren nach einem der Ansprüche 1 bis 4, **dadurch gekennzeichnet, dass** zur Erkennung eines fehlerhaften Gefäßzugangs die Differenz von dem venösen und arteriellen Differenzdruck und der Testfunktion bestimmt wird, wobei auf einen fehlerhaften Gefäßzugang geschlossen wird, wenn die Differenz von dem Differenzdruck und der Testfunktion größer als ein vorgegebener Grenzwert ist.

**6.** Verfahren nach einem der Ansprüche 1 bis 5, **dadurch gekennzeichnet, dass** die Störungen des arteriellen und venösen Drucksignals beschreibende Testfunktion aus einer Linearkombination von trigonometrischen Funktionen ermittelt wird.

**7.** Verfahren nach Anspruch 6, **dadurch gekennzeichnet, dass** die Störungen des arteriellen und venösen Drucksignals beschreibende Testfunktion durch folgende Gleichung beschrieben wird:

$$\hat{P}_{VA}(t)=B_0+\sum_{k=1}^{K}\sum_{n=1}^{N}\left[A_{nk}\sin(n\omega_k t)+B_{nk}\cos(n\omega_k t)\right]$$

wobei die Koeffizienten A und B die Beiträge der höheren Harmonischen der Ordnung n der periodischen Störung $k$ mit der Frequenz $w_k$ bestimmen.

**8.** Vorrichtung zur Überwachung eines Gefäßzugangs für eine extrakorporale Blutbehandlungsvorrichtung zur Durchführung einer extrakorporalen Blutbehandlung, bei der Blut aus dem Gefäßzugang über einen arteriellen Zweig eines extrakorporalen Blutkreislaufs in ein Blutbehandlungselement strömt und aus dem Blutbehandlungselement über einen venösen Zweig zurück in den Gefäßzugang strömt, mit
einer Messeinheit (18, 19) zum Messen des Drucks im venösen und arteriellen Zweig des extrakorporalen Blutkreislaufs und
einer Auswerteinheit (22) zum Auswerten des gemessenen venösen und arteriellen Druck zur Feststellung eines fehlerhaften Gefäßzugangs,
**dadurch gekennzeichnet,**
**dass** die Auswerteinheit (22) Mittel zum Ermitteln einer Störungen des gemessenen arteriellen und venösen Drucks beschreibenden Testfunktion aufweist, wobei die Auswerteinheit (22) derart ausgebildet ist, dass mit der Störungen des arteriellen und venösen Druck beschreibenden Testfunktion ein von Störungen befreiter Differenzdruck aus dem gemessenen venösen und arteriellen Druck ermittelt wird, wobei der von Störungen befreite Differenzdruck zur Erkennung eines fehlerhaften Gefäßzugangs ausgewertet wird.

**9.** Vorrichtung nach Anspruch 8,

**dadurch gekennzeichnet, dass** die
Auswerteinheit (22) derart ausgebildet ist, dass zur Ermittlung des von Störungen befreiten Differenzdrucks die Differenz des venösen und arteriellen Drucks gebildet wird und dass nach der Differenzbildung der Differenzdruck mit der Störungen des arteriellen und venösen Drucks beschreibenden Testfunktion von Störungen befreit wird.

**10.** Vorrichtung nach Anspruch 9, **dadurch gekennzeichnet, dass** die Auswerteinheit (22) derart ausgebildet ist, dass die Testfunktion in einem vorausgehenden Zeitintervall ermittelt wird, und dass der gemessene Differenzdruck in einem nachfolgenden Zeitintervall mit der in dem vorausgehenden Zeitintervall ermittelten Testfunktion verglichen wird, wobei die in dem vorausgehenden Zeitintervall ermittelte Testfunktion als eine Störungen des arteriellen und venösen Drucksignals beschreibende Schätzfunktion für das nachfolgende Zeitintervall angenommen wird.

**11.** Vorrichtung nach Anspruch 10, **dadurch gekennzeichnet, dass** die Auswerteinheit (22) derart ausgebildet ist, dass die Testfunktion während der extrakorporalen Blutbehandlung sukzessive ermittelt wird.

**12.** Vorrichtung nach einem der Ansprüche 8 bis 11, **dadurch gekennzeichnet, dass** die Auswerteinheit (22) derart ausgebildet ist, dass zur Erkennung eines fehlerhaften Gefäßzugangs die Differenz von dem venösen und arteriellen Differenzdruck und der Testfunktion bestimmt wird, wobei auf einen fehlerhaften Gefäßzugang geschlossen wird, wenn die Differenz von dem Differenzdruck und der Testfunktion größer als ein vorgegebener Grenzwert ist.

**13.** Vorrichtung nach einem der Ansprüche 8 bis 12, **dadurch gekennzeichnet, dass** die Auswerteinheit (22) derart ausgebildet ist, dass die Störungen des arteriellen und venösen Drucksignals beschreibende Testfunktion aus einer Linearkombination von trigonometrischen Funktionen ermittelt wird.

**14.** Vorrichtung nach Anspruch 13, **dadurch gekennzeichnet, dass** die Auswerteinheit (22) derart ausgebildet ist, dass die Störungen des arteriellen und venösen Drucksignals beschreibende Testfunktion durch folgende Gleichung beschrieben wird:

$$\hat{P}_{VA}(t)=B_0+\sum_{k=1}^{K}\sum_{n=1}^{N}\left[A_{nk}\sin(n\omega_k t)+B_{nk}\cos(n\omega_k t)\right]$$

wobei die Koeffizienten A und B die Beiträge der höheren Harmonischen der Ordnung n der periodischen Störung $k$ mit der Frequenz $w_k$ bestimmen.

**15.** Vorrichtung zur extrakorporalen Blutbehandlung mit einer Vorrichtung zur Überwachung eines Gefäßzugangs nach einem der Ansprüche 8 bis 14.

## Claims

**1.** Method of monitoring a vascular access during extracorporeal blood treatment in which blood flows from the vascular access, via an arterial segment of an extra-corporeal blood circuit, into a blood-treating element and from the blood-treating element, via a venous segment, back into the vascular access, the pressure in the venous and arterial segments of the extracorporeal blood circuit being measured and the venous and arterial pressures which are measured being analysed to allow a faulty vascular access to be found,
**characterised in that**
a test function which defines interference affecting the arterial and venous pressures which are measured is determined, and
**in that** a pressure differential which is freed from interference is determined from the venous and arterial pressures which are measured using the test function which defines interference affecting the arterial and venous pressures, and **in that** the pressure differential which has been freed from interference is analysed to allow a faulty vascular access to be detected.

**2.** Method according to claim 1, **characterised in that**, to allow the pressure differential freed from interference to be determined, the difference between the venous and arterial pressures is formed and, the difference having been formed, the pressure differential is freed from interference using the test function which defines interference affecting the arterial and venous pressures.

3. Method according to claim 2, **characterised in that** the test function is determined in a preceding interval of time, and **in that** the pressure differential measured is compared, in a subsequent interval of time, with the test function which was determined in the preceding interval of time, the test function determined in the preceding interval of time being taken as an estimated function which defines interference affecting the arterial and venous pressures for the subsequent interval of time.

4. Method according to claim 3, **characterised in that** the test function is determined successively during the extra-corporeal blood treatment.

5. Method according to one of claims 1 to 4, **characterised in that**, to allow a faulty vascular access to be detected, the difference is determined between the venous and arterial pressure differential and the test function, the conclusion that there is a faulty vascular access being drawn if the difference between the pressure differential and the test function is more than a preset limiting value.

6. Method according to one of claims 1 to 5, **characterised in that** the test function which defines the interference affecting the arterial and venous pressure signals is determined from a linear combination of trigonometrical functions.

7. Method according to claim 6, **characterised in that** the test function which defines interference affecting the venous and arterial pressure signals is defined by the following equation:

$$\hat{P}_{VA}(t) = B_0 + \sum_{k=1}^{K} \sum_{n=1}^{N} \left[A_{nk} \sin(n\omega_k t) + B_{nk} \cos(n\omega_k t)\right]$$

where the coefficients A and B determine the contributions made by the higher harmonics of order n of the cyclic interference k of frequency $\omega_k$.

8. Arrangement for monitoring a vascular access for an extracorporeal blood-treating apparatus for performing extra-corporeal blood treatment in which blood flows from the vascular access, via an arterial segment of an extra-corporeal blood circuit, into a blood-treating element and from the blood-treating element, via a venous segment, back into the vascular access, having
a measuring unit (18, 19) for measuring the pressure in the venous and arterial segments of the extracorporeal blood circuit and
an analysing unit (22) for analysing the venous and arterial pressures which are measured to enable a faulty vascular access to be found,
**characterised in that**
the analysing unit (22) has means for determining a test function which defines interference affecting the arterial and venous pressures which are measured, the analysing unit (22) being so designed that a pressure differential which is freed from interference is determined from the venous and arterial pressures which are measured, using the test function which defines interference affecting the arterial and venous pressures, the pressure differential which is freed from interference being analysed to allow a faulty access to be detected.

9. Arrangement according to claim 8, **characterised in that** the analysing unit (22) is so designed that, to allow the pressure differential freed from interference to be determined, the difference between the venous and arterial pressures is formed and **in that**, the difference having been formed, the pressure differential is freed from interference using the test function which defines interference affecting the arterial and venous pressures.

10. Arrangement according to claim 9, **characterised in that** the analysing unit (22) is so designed that the test function is determined in a preceding interval of time, and **in that**, in a subsequent interval of time, the pressure differential measured is compared with the test function which was determined in the preceding interval of time, the test function determined in the preceding interval of time being taken as an estimated function which defines interference affecting the arterial and venous pressure signals for the subsequent interval of time.

11. Arrangement according to claim 10, **characterised in that** the analysing unit (22) is so designed that the test function is determined successively during the extra-corporeal blood treatment.

12. Arrangement according to one of claims 8 to 11, **characterised in that** the analysing unit (22) is so designed that, to allow a faulty vascular access to be detected, the difference is determined between the venous and arterial

pressure differential and the test function, the conclusion that there is a faulty vascular access being drawn if the difference between the pressure differential and the test function is more than a preset limiting value.

13. Arrangement according to one of claims 8 to 12, **characterised in that** the analysing unit (22) is so designed that the test function which defines interference affecting the arterial and venous pressure signals is determined from a linear combination of trigonometrical functions.

14. Arrangement according to claim 13, **characterised in that** the analysing unit (22) is so designed that the test function which defines interference affecting the arterial and venous pressure signals is defined by the following equation:

$$\hat{P}_{VA}(t) = B_0 + \sum_{k=1}^{K} \sum_{n=1}^{N} \left[A_{nk} \sin(n\omega_k t) + B_{nk} \cos(n\omega_k t)\right]$$

where the coefficients A and B determine the contributions made by the higher harmonics of order n of the cyclic interference k of frequency $\omega_k$.

15. Apparatus for extracorporeal blood treatment having an arrangement for monitoring a vascular access according to one of claims 8 to 14.

**Revendications**

1. Procédé de surveillance d'un accès vasculaire pendant un traitement sanguin extracorporel, dans lequel du sang de l'accès vasculaire entre par une branche artérielle d'un circuit sanguin extracorporel dans un élément de traitement sanguin et sort de l'élément de traitement sanguin par une branche veineuse pour revenir dans l'accès vasculaire, dans lequel la pression dans la branche veineuse et artérielle du circuit sanguin extracorporel est mesurée et la pression veineuse et artérielle mesurée est évaluée pour déterminer un accès vasculaire défectueux,
**caractérisé en ce que**
une fonction de test décrivant des troubles de la pression artérielle et veineuse mesurée est déterminée et
avec la fonction de test décrivant des troubles de la pression artérielle et veineuse mesurée est déterminée une pression différentielle exempte de troubles de la pression veineuse et artérielle mesurée, et
la pression différentielle exempte de troubles est analysée pour la reconnaissance d'un accès vasculaire défectueux.

2. Procédé selon la revendication 1, **caractérisé en ce que** pour la détermination de la pression différentielle exempte de troubles, la différence de la pression veineuse et artérielle est formée et **en ce que**, après la formation de la différence, la pression différentielle avec la fonction de test décrivant des troubles de la pression artérielle et veineuse est exempte de troubles.

3. Procédé selon la revendication 2, **caractérisé en ce que** la fonction de test est déterminée dans un intervalle temporel antérieur, et **en ce que** la pression différentielle mesurée est comparée, dans un intervalle temporel consécutif, à la fonction de test déterminée dans l'intervalle temporel antérieur, dans lequel la fonction de test déterminée dans l'intervalle temporel antérieur est adoptée en tant que fonction d'évaluation décrivant des troubles du signal de pression artérielle et veineuse pour l'intervalle temporel consécutif.

4. Procédé selon la revendication 3, **caractérisé en ce que** la fonction de test est déterminée de façon successive pendant le traitement sanguin extracorporel.

5. Procédé selon l'une des revendications 1 à 4, **caractérisé en ce que**, pour la reconnaissance d'un accès vasculaire défectueux, la différence de la pression différentielle veineuse et artérielle et de la fonction de test est déterminée, dans lequel on conclut à un accès vasculaire défectueux si la différence de la pression différentielle et de la fonction de test est plus grande qu'une valeur seuil prédéterminée.

6. Procédé selon l'une des revendications 1 à 5, **caractérisé en ce que** la fonction de test décrivant des troubles du signal de pression artérielle et veineuse est déterminée à partir d'une combinaison linéaire de fonctions trigonométriques.

7. Procédé selon la revendication 6, **caractérisé en ce que** la fonction de test décrivant des troubles du signal de pression artérielle et veineuse est décrite par l'équation suivante :

$$\hat{P}_{VA}(t)=B_0+\sum_{k=1}^{K}\sum_{n=1}^{N}\left[A_{nk}\sin(n\omega_k t)+B_{nk}\cos(n\omega_k t)\right]$$

dans laquelle les coefficients A et B déterminent les contributions des harmoniques supérieures d'ordre n du trouble périodique *k* avec la fréquence $w_k$.

8. Dispositif de surveillance d'un accès vasculaire pour un dispositif de traitement sanguin extracorporel pour la réalisation d'un traitement sanguin extracorporel, dans lequel du sang de l'accès vasculaire entre par une branche artérielle d'un circuit sanguin extracorporel dans un élément de traitement sanguin et sort de l'élément de traitement sanguin par une branche veineuse pour revenir dans l'accès vasculaire, avec
une unité de mesure (18, 19) pour mesurer la pression dans la branche veineuse et artérielle du circuit sanguin extracorporel et
une unité d'analyse (22) pour analyser la pression veineuse et artérielle mesurée pour déterminer un accès vasculaire défectueux,
**caractérisé en ce que**,
l'unité d'analyse (22) présente un moyen de détermination d'une fonction de test décrivant des troubles de la pression artérielle et veineuse mesurée, dans lequel l'unité d'analyse (22) est réalisée de telle sorte qu'avec la fonction de test décrivant des troubles de la pression artérielle et veineuse est déterminée une pression différentielle exempte de troubles à partir de la pression veineuse et artérielle mesurée, dans lequel la pression différentielle exempte de troubles est analysée pour la reconnaissance d'un accès vasculaire défectueux.

9. Dispositif selon la revendication 8,
**caractérisé en ce que**
l'unité d'analyse (22) est réalisée de telle sorte que, pour la détermination de la pression différentielle exempte de troubles, la différence de la pression veineuse et artérielle est formée et après la formation de la différence, la pression différentielle avec la fonction de test décrivant des troubles de la pression artérielle et veineuse est exempte de troubles.

10. Dispositif selon la revendication 9, **caractérisé en ce que** l'unité d'analyse (22) est réalisée de telle sorte que la fonction de test est déterminée dans un intervalle temporel antérieur, et **en ce que** la pression différentielle mesurée est comparée, dans un intervalle temporel consécutif, à la fonction de test déterminée dans l'intervalle temporel antérieur, dans lequel la fonction de test déterminée dans l'intervalle temporel antérieur est adoptée en tant que fonction d'évaluation décrivant des troubles du signal de pression artérielle et veineuse pour l'intervalle temporel consécutif.

11. Procédé selon la revendication 10, **caractérisé en ce que** l'unité d'analyse (22) est réalisée de telle sorte que la fonction de test est déterminée de façon successive pendant le traitement sanguin extracorporel.

12. Dispositif selon l'une des revendications 8 à 11, **caractérisé en ce que** l'unité d'analyse (22) est réalisée de telle sorte que, pour la reconnaissance d'un accès vasculaire défectueux, la différence de la pression différentielle veineuse et artérielle et de la fonction de test est déterminée, dans lequel on conclut à un accès vasculaire défectueux si la différence de la pression différentielle et de la fonction de test est plus grande qu'une valeur seuil prédéterminée.

13. Dispositif selon l'une des revendications 8 à 12, **caractérisé en ce que** l'unité d'analyse (22) est réalisée de telle sorte que la fonction de test décrivant des troubles du signal de pression artérielle et veineuse est déterminée à partir d'une combinaison linéaire de fonctions trigonométriques.

14. Dispositif selon la revendication 13, **caractérisé en ce que** l'unité d'analyse (22) est réalisée de telle sorte la fonction de test décrivant des troubles du signal de pression artérielle et veineuse est décrite par l'équation suivante :

$$\hat{P}_{VA}(t)=B_0+\sum_{k=1}^{K}\sum_{n=1}^{N}\left[A_{nk}\sin(n\omega_k t)+B_{nk}\cos(n\omega_k t)\right]$$

dans laquelle les coefficients A et B déterminent les contributions des harmoniques supérieures d'ordre n du trouble périodique *k* avec la fréquence $w_k$.

**15.** Dispositif de traitement sanguin extracorporel avec un dispositif de surveillance d'un accès vasculaire selon l'une des revendications 8 à 14.

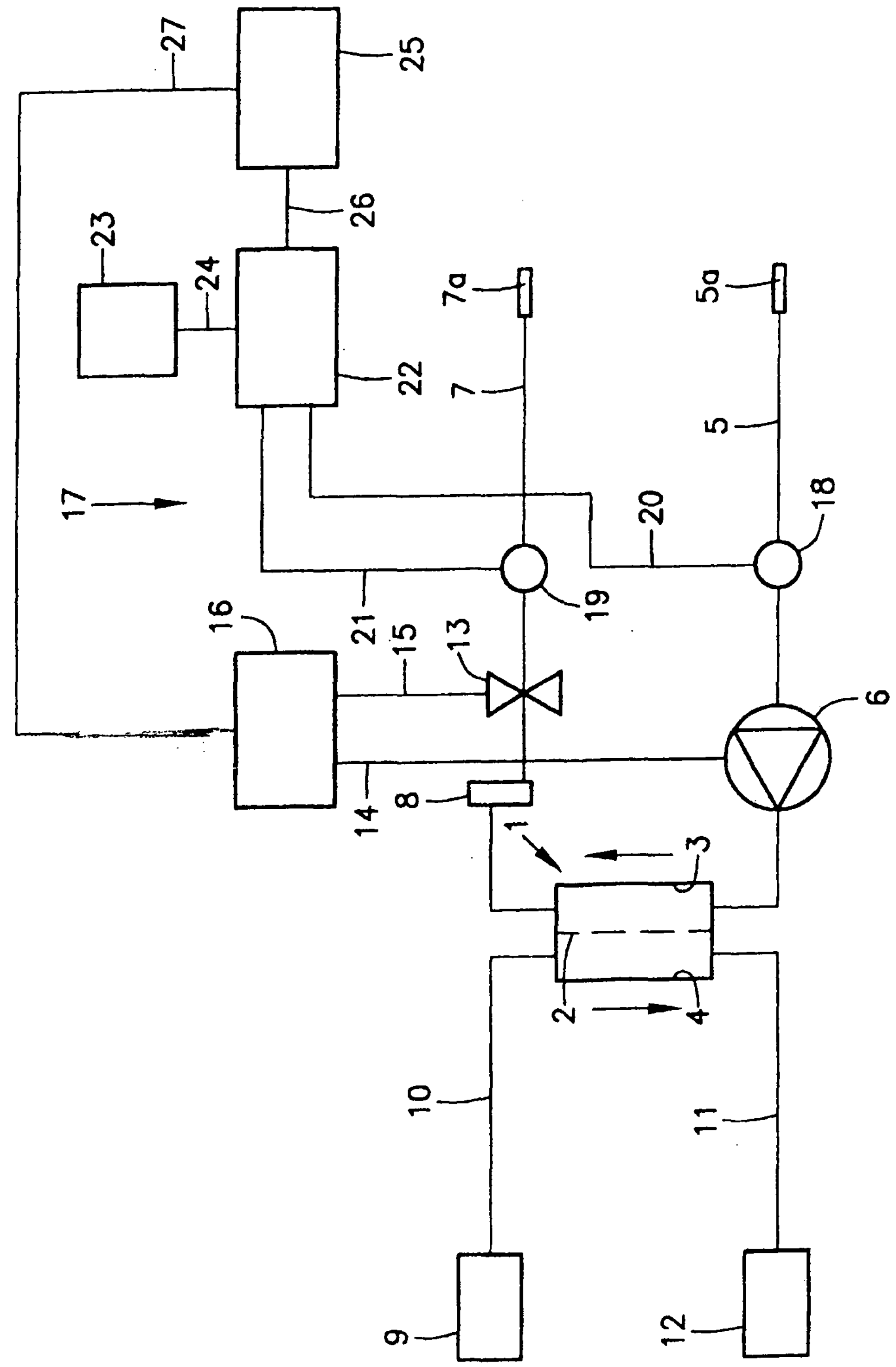


Fig. 1

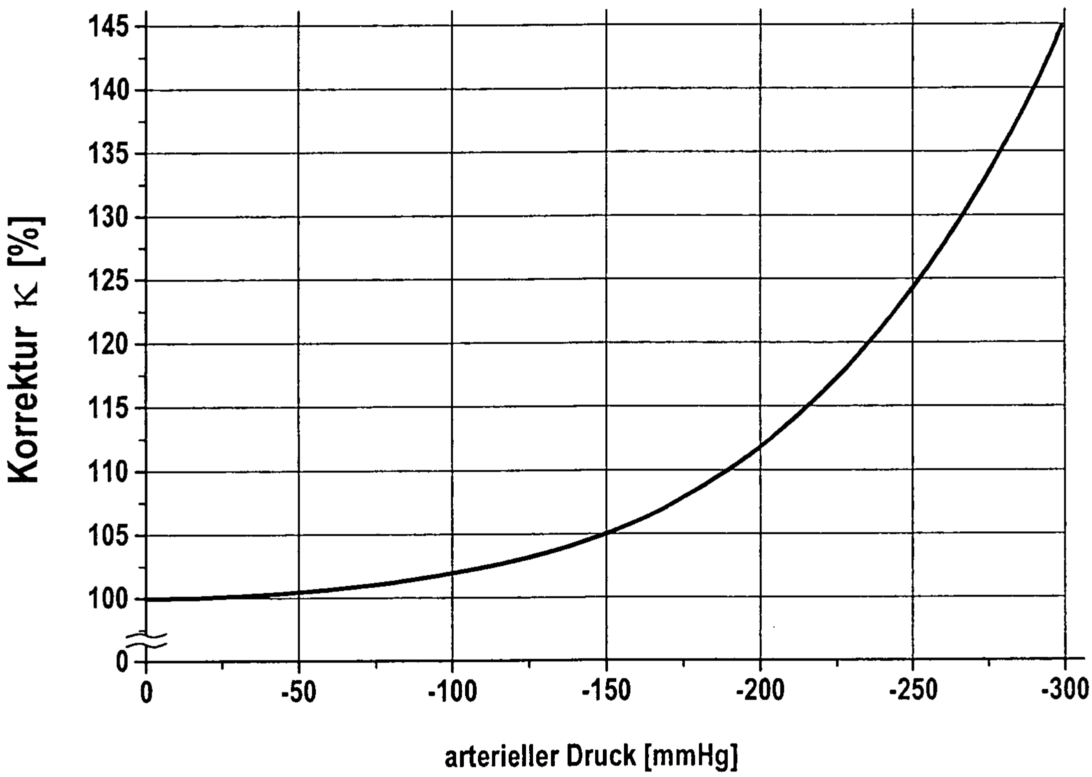
Korrektur κ [%]
145
140
135
130
125
120
115
110
105
100
0
0
-50
-100
-150
-200
-250
-300
arterieller Druck [mmHg]

Fig. 2

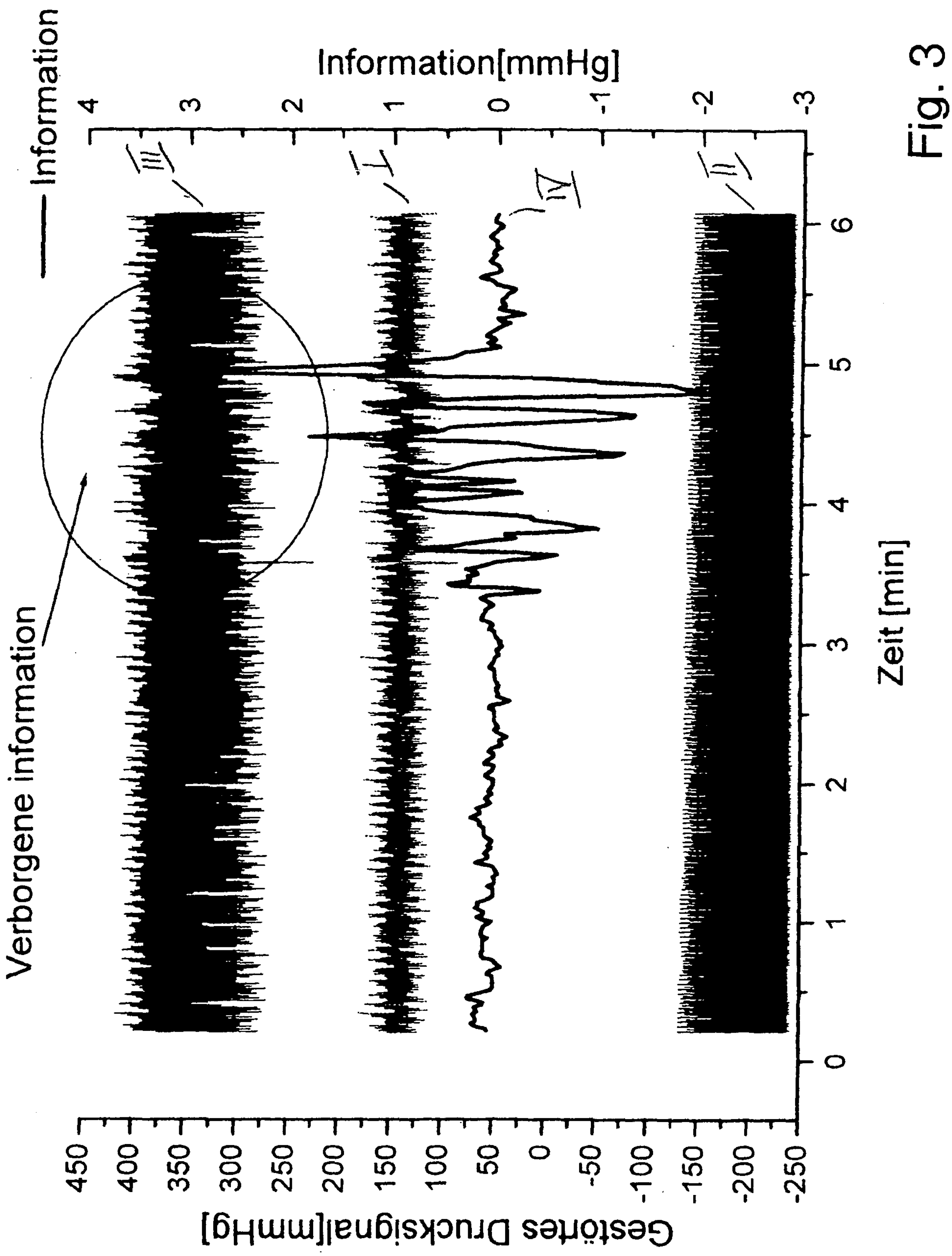
Information
Information[mmHg]
Verborgene information
Gestörtes Drucksignal[mmHg]
Zeit [min]
I
II
III
IV

Fig. 3

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 6221040 B1
- DE 10115991 C1
- US 20020174721 A1
- DE 102006032815 A1